# EUROPEAN PATENT APPLICATION

(11) **EP 2 533 048 A1**
(43) Date of publication of application: **12.12.2012**
(21) Application number: 11739788.5
(22) Date of filing: 02.02.2011
(51) Int. Cl.: G01N 33/53, G01N 33/15, G01N 33/50

(54) **COMPOUND CAPABLE OF BINDING TO NATURALLY OCCURRING DENATURED PROTEIN, AND METHOD FOR SCREENING FOR THE COMPOUND**

(30) Priority: 03.02.2010 JP 2010022622
(71) Applicant: PRISM BioLab Co., Ltd., Yokohama-shi, Kanagawa 226-8510 (JP)
(72) Inventor: KOUJI, Hiroyuki, Yokohama-shi Kanagawa 226-8510 (JP)
(74) Representative: Webster, Jeremy Mark
(86) International application number: PCT/JP2011/052158
(87) International publication number: WO 2011/096440

(57) **Abstract**

Screening for low molecular weight compounds that regulate protein-protein interactions of a large number of proteins, including transcription factors, that have disordered sequences has been performed, but it has been almost impossible to obtain low molecular weight compounds having sufficient activity. The present inventors found that, regarding screening for a protein that regulates an interaction between an intrinsically disordered protein and a partner protein, carrying out screening while focusing on an disordered sequence, and selecting a candidate compound from peptidomimetic compounds that have a peptidomimetic backbone and have a peptide side chain or a side chain similar thereto enable a compound that regulates the activity of an intrinsically disordered protein to be efficiently selected, and the present inventors accomplished the present invention.

## Description

### [Technical Field]

The present invention relates to a compound that binds to a disordered sequence region (hereinafter sometimes referred to as a disordered region) of an intrinsically disordered protein. Specifically, the present invention relates to a compound that can bind to a disordered sequence region of an intrinsically disordered protein, alter the structure of the disordered sequence domain due to the binding, making binding to a partner protein, which should occur in a natural state of the intrinsically disordered protein, impossible, and thus inhibit a biological reaction that occurs in a natural state. Also, the present invention relates to, for example, a screening method for a compound that binds to a disordered sequence region of an intrinsically disordered protein. Furthermore, the present invention relates to a pharmaceutical containing a compound that binds to a disordered sequence region of an intrinsically disordered protein.

### [Background Art]

It has been said so far that proteins that function in vivo form specific conformations and demonstrate specific functions based on such conformations. In recent years, however, contrary to such a generally-accepted idea, it is known that there are a large number of proteins in vivo that do not form any conformation and there are proteins part of which does not form any conformation. It is not uncommon in a protein that an amino acid sequence portion that does not form any conformation extends as many as a few hundred residues. Such a sequence portion is referred to as an "intrinsically disordered" region, and a protein molecule that is entirely composed of a disordered region or a protein that has, even if partially, a disordered region are referred to as intrinsically disordered proteins.

Since the research conducted by an American biochemist Christian B. Anfinsen, an idea that "a primary structure determines a higher-order structure" has been established. This is called the "Anfinsen's dogma", and a scheme: "primary structure → specific conformation → specific function of a protein" has been established. In other words, there is a tacit understanding that a protein lacking a specific conformation (= denatured state) → a (denatured) protein lacking in function.

For the past ten years, however, it has come to be known that there are proteins that have long disordered regions, i.e., intrinsically disordered proteins (IDPs). IDPs have features such as:
(1) A large number of IDPs are present in eukaryotic cells (many are present particularly in nuclei) and few in prokaryotic cells;
(2) IDPs have a large amount of hydrophilicity residue and a small amount of hydrophobic region;
(3) Many IDPs bind to other proteins, for example, transcription factors, coactivators such as CBPs, intracellular signaling proteins, cell cycle control factors such as p53, cellular membrane fusion factors, RNA binding proteins, and the like to demonstrate their functions; and
(4) the function of such proteins are closely associated with disordered regions.

Research conducted so far has revealed that IDPs are ubiquitously present in almost all organisms and associated with various biological functions. It is said that about 20% of the proteins produced by eukaryotic cells are IDPs, and disordered regions extend as many as 50 to 500 residues. IDPs usually have disordered sequences that do not have higher-order structures, but once IDPs bind to target proteins, such structures are induced, and IDPs demonstrate functions.

In general, a number of transcription factors have disordered regions. Transcription factors are proteins that control the initiation and repression of the transcription process in gene expression, and there are many kinds of transcription factors depending on the combination with the genes that receive control. It is said that there are more than 1000 kinds of transcription factor genes in the human genome. An IDP prediction from amino acid sequences using a DISODRED program (IDP prediction program) has predicted that, on average, as much as half the full length (49%) is a disordered region in a human transcription factor.

Heretofore, it has been a fact that a large number of intrinsically disordered proteins are present in vivo, and it has been said that a large number of intrinsically disordered proteins are present particularly among important proteins that are involved in the signal transduction system, gene expression control, and the like. On the other hand, however, due to the fact that intrinsically disordered proteins have been attracting attention rather recently, there are still a large number of unclear points about intrinsically disordered proteins.
As a matter of fact, proteins that do not form any conformation or that have a portion not forming any conformation have been known from before. However, no screening has been carried out with a focus on a disordered sequence.

### [Citation List]

### [Non Patent Literature]

[NPL 1] Seibutsu Butsuri 49(1), 004-010 (2009)
[NPL 2] Proc NatlAcad Sci USA. 2004 Aug 24; 101(34): 12682-7

### [Summary of Invention]

### [Technical Problem]

Screening for low molecular weight compounds that regulate protein-protein interactions of a large number of proteins, including transcription factors, that have disordered sequences has been performed, but it has been almost impossible to obtain low molecular weight compounds having sufficient activity. Accordingly, an object of the present invention is to provide a screening method for obtaining an activity regulating agent for an intrinsically disordered protein or an activity regulating agent for binding between an intrinsically disordered protein and a partner protein thereof. Also, an object of the present invention is to provide a compound obtained by such a screening method, and a pharmaceutical agent containing such a compound.

### [Solution to Problem]

When superimposed on a structural domain and a disordered region, the position of a functional site revealed by a biochemical method was located not in the structural domain but rather in the disordered region in almost all cases. This means that a disordered region of a protein can be responsible for a biologically important function. Therefore, it is possible to think that controlling IDPs is an attractive technique for therapeutic agents for various diseases.

Such substances are very useful as therapeutic agents for diseases, and since a disordered sequence has a domain structure, selecting a substance that binds domain-selectively may possibly give an ideal pharmaceutical agent that is selective and side effect-free. Moreover, as described above, since a disordered sequence is mainly present in a cell, an intracellular protein-protein interaction can be controlled, which is not possible with conventional pharmaceutical agents, and thus making it possible to produce therapeutic agents for so-called intractable diseases.

Accordingly, having conducted diligent research, the present inventors found that, in screening for an activity regulating agent for an intrinsically disordered protein or an activity regulating agent for binding between an intrinsically disordered protein and a partner protein thereof, carrying out screening while focusing attention on an interaction in a disordered sequence of the intrinsically disordered protein enables efficient screening for a compound that regulates the activity of the intrinsically disordered protein, particularly through the use of a peptidomimetic compound that has a peptidomimetic backbone and that has a peptide side chain or a side chain similar thereto as a test substance.
It seems that, when peptidomimetic compounds that have a peptidomimetic backbone and that have a peptide side chain or a side chain similar thereto and disordered regions of intrinsically disordered proteins demonstrate an interaction, the disordered regions efficiently arrive at a state similar to a modified random coil. If so, questions arise as to why they do not associate or aggregate with each other in vivo, or why they are not decomposed by various proteases (degrading enzymes). But, appearing to go against the generally accepted ideas as described above may be the greatest feature of intrinsically disordered proteins.

That is, the present invention provides a screening method, a compound, an activity regulating agent for an intrinsically disordered protein, an activity regulating method for an intrinsically disordered protein, and a pharmaceutical as follows:
[1] A method for screening for an activity regulating agent for an intrinsically disordered protein, including the steps of:
   (1) bringing the protein or a disordered sequence region of the protein into contact with a test substance, and
   (2) detecting an interaction between the disordered sequence region of the protein and the test substance.
[2] A method for screening for an activity regulating agent for binding between an intrinsically disordered protein and a partner protein thereof, including the steps of:
   (1) bringing the protein or a disordered sequence region of the protein into contact with a test substance, and
   (2) detecting an interaction between the disordered sequence region of the protein and the test substance.
[3] A method for screening for an activity regulating agent for binding between an intrinsically disordered protein and a partner protein thereof, including the steps of:
   (1) bringing the protein or a disordered sequence region of the protein, the partner protein, and a test substance into contact with each other, and
   (2) detecting an interaction between the disordered sequence region of the protein and the test substance.
[4] A method for screening for an activity regulating agent for binding between an intrinsically disordered protein and a partner protein thereof, including the steps of:
   (1) bringing the protein or a disordered sequence region of the protein, the partner protein, and a test substance into contact with each other, and
   (2) detecting an interaction between the disordered sequence region of the protein and the partner protein.
[5] The screening method according to items [1] to [4], wherein the test substance is a peptidomimetic compound.
[6] The screening method according to items [1] to [5], wherein the intrinsically disordered protein is a transcription regulatory factor, a protein of a signal transduction system, or an intracellular protein.
[7] The screening method according to items [1] to [6], wherein detection of interaction is carried out according to a two-hybrid method (Y2H), co-immunoprecipitation, a protein chip method, a conformational analysis, far-Western blotting, a crosslinking method, or fluorescence quenching.
[8] A compound obtained by a screening method of items [1] to [8].
[9] A compound that binds to a disordered sequence region of an intrinsically disordered protein.
[10] An activity regulating agent for an intrinsically disordered protein, containing a compound that binds to a disordered sequence region of an intrinsically disordered protein.
[11] A method for regulating activity of an intrinsically disordered protein using a compound that binds to a disordered sequence region of the intrinsically disordered protein.
[12] A pharmaceutical containing a compound that binds to a disordered sequence region of an intrinsically disordered protein.

### [Brief Description of Drawings]

Fig. 1 shows data obtained when ICG-001 and CBP were pulled down according to immunoprecipitation. This experiment revealed that ICG-001 is bound to CBP.
Fig. 2 shows results of an experiment indicating that ICG-001 is bound to the N-terminus of CBP. Fig. 2 is experiment data showing that when CBP (1-111) and ß-catenin expressed in E. coli were pulled down according to immunoprecipitation, CBP (1-111) became separated due to the addition of ICG-001, indicating that binding to P300, which is highly analogous to CBP, does not occur.
Fig. 3 is a diagram showing the structure of CBP. Circular portions indicate ordered sequences and linear portions indicate disordered sequences (P. E. Wright, Nature Review 2009).
Fig. 4 shows results of an NMR analysis using CBP (1-111) expressed in E. coli as a sample, obtained as a 2D spectrum with the X axis indicating C-13 data and the Y axis indicating N-15 data.
Fig. 5 shows results of an NMR analysis using a sample in which CBP (1-111) expressed in E. coli and 5 µM of ICG-001 were concomitantly present, obtained as a 2D spectrum, with the X axis indicating C-13 data and the Y axis indicating N-15 data.
Fig. 6 shows results of an NMR analysis using a sample in which CBP (1-111) expressed in E. coli and 15 µM of ICG-001 were concomitantly present, obtained as a 2D spectrum, with the X axis indicating C-13 data and the Y axis indicating N-15 data.

### [Description of Embodiments]

The present invention will now be described in detail below. Herein, genetic engineering techniques can be performed in accordance with known techniques as described in, for example, "Molecular Cloning" (Sambrook, J. et al., Cold Spring Harbor Laboratory Press, 1989) unless specified otherwise, and protein engineering techniques can be performed in accordance with known techniques as described in, for example, "Tanpaku Jikken Protocol (protein experimental protocol)" (Shujunsha, 1997) unless specified otherwise.
The intrinsically disordered protein may be any protein insofar as it has a disordered sequence as described above. The intrinsically disordered protein may be a protein that is entirely composed of a disordered sequence, or may be a protein that partially contains a disordered sequence in, for example, an N-terminus region, a C-terminus region, or an intermediate region. It may be a combination thereof. Examples thereof include transcription regulatory factors, proteins of the signal transduction system, and other intracellular functional proteins having a disordered sequence. Specific examples include transcription regulatory factors such as CBP, c-Myc, Fos, SRF, EGR-4, c-Myb, and TBX3 composed solely of a DNA binding domain and a disordered sequence; transcription regulatory factors such as p53, IRF-3, E2F-1, Ets-1, HIF-1α, AR, NF-AT1, and SREBP-1 having a DNA binding domain, another domain, and a disordered sequence; and the like.

The partner protein encompasses any protein that interacts with the aforementioned intrinsically disordered proteins. For example, in the case where the intrinsically disordered protein is CBP, examples of partner proteins that shows an interaction include HIF-1, SYT, p53, p73, Mdm2, Stat-2, TAL-1, Ets-1, TBP, NF-kB, HNF-4, CREB, SREBP, c-JUN, c-Myb, ATF, BRCA-1, Sap1, NF-E2, TFIIB, P/CAF, MyoD, c-Fos, Ets-1, C/EBP, E2F, GATA-1, MI, SYT, p53, Smad, SRC-1, p/CIP, and YY1.

When the intrinsically disordered protein is used for screening, the intrinsically disordered protein may be used in a naturally occurring state, or the disordered portion may be cut out and used. The disordered portion may take any form insofar as an interaction with a test substance can be detected, and the disordered sequence portion and a portion having a domain structure may be bound to each other.

Examples of test substances include low molecular weight compounds, peptides, proteins, peptidomimetic compounds, nucleic acids, nucleic acid analogous compounds, cholesterols; retinoic acid, prostaglandin, and like biologically active substances; and derivatives and analogs thereof. Preferable examples include peptidomimetic compounds, and more specific examples include α-helix mimetic compounds, β-sheet mimetic compounds, and β-turn mimetic compounds. Peptidomimetic compounds are, for example, compounds in which the backbone thereof has a spatial structure highly similar to α-helix, β-sheet, and/or β-turn, and the side chain thereof projects in a direction highly similar to that of a peptide and is preferably an amino acid side chain or a side chain having a similar structure.

It is sufficient that the backbone of the peptidomimetic compound does not have a readily decomposable structure such as a peptide bond and has a similar pharmacophore, and examples include those presented in WO92/13878, WO94/03494, WO95/00534, WO95/25120, WO96/30035, WO96/30396, WO98/05333, WO97/15577, WO98/49168 WO01/00210, WO97/15589, WO02/092010, WO2003/030907, WO2003/031448, WO2004/093828, WO2005/116032, WO2004/108731, WO2006/101858, WO2007/056513, WO2007/056593, WO2007/062243, WO2007/139346, WO2009/051397, WO2009/051398, WO2009/051399, WO2010/027114, and WO2010/120112. Apart from such compounds, those having a similar backbone are preferable as test substances.

In the present invention, screening for an activity regulating agent for an intrinsically disordered protein is carried out by (1) bringing the intrinsically disordered protein or a disordered sequence region of the protein into contact with a test substance, and (2) detecting an interaction (i.e., intermolecular binding) between the disordered sequence region of the protein and the test substance.
In the present invention, screening for an activity regulating agent for binding between an intrinsically disordered protein and a partner protein thereof is carried out by (1) bringing the intrinsically disordered protein or a disordered sequence region of the protein into contact with a test substance, or bringing the intrinsically disordered protein or a disordered sequence region of the protein, a partner protein, and a test substance into contact with each other, and (2) detecting an interaction (i.e., intermolecular binding) between the disordered sequence region of the intrinsically disordered protein and the test substance, or detecting an interaction (i.e., intermolecular binding) between the disordered sequence region of the intrinsically disordered protein and the partner protein.
The method for detecting an interaction between a disordered sequence region of an intrinsically disordered protein and a test substance is not particularly limited insofar as the method can detect an interaction between the disordered sequence region and the test substance. Also, an effect imparted by the test substance to the interaction between the intrinsically disordered protein and the partner protein that interacts therewith may be detected. In any case, an interaction is not merely detected, and it is preferable to detect an interaction while focusing attention on the disordered region of the intrinsically disordered protein. Examples of such detection methods include a two-hybrid method (Y2H), co-immunoprecipitation, a protein chip method, a conformational analysis, far-Western blotting, a crosslinking method, fluorescence quenching, and the like.

Such methods are known methods, and the two-hybrid method (Y2H) is a method that takes advantage of activity exhibited only after two molecules bind to each other. An example of a yeast two-hybrid system will now be presented to describe the principle of the two-hybrid system. The yeast two-hybrid system takes advantage of characteristics of various eukaryotic transcription factors that possess two separable functional domains. One of the two domains is a DNA binding domain (sometimes abbreviated as a "DBD") that specifically recognizes and binds to a cis element, and the other is a transcription activation domain (sometimes abbreviated as an "AD") that activates transcription. In the two-hybrid system, a so-called bait protein containing a DNA binding domain (GAL4bd or lexA) and a protein "A" of interest is expressed as a fusion protein in a yeast. Simultaneously, the same yeast cell expresses a so-called fish protein containing a DNA activation domain (GAL4ad or VP16) and a protein "B". When the bait protein and the fish protein interact, the DNA binding domain and the transcription activation domain of the fusion proteins come close, and the resulting protein complex induces expression of a reporter gene (for example, HIS3 or lacZ). This expression can be readily monitored by culturing the yeast cell on a histidine-free selective medium or by activation of a lacZ gene. For example, a DNA sequence that encodes an unknown fish protein can be readily identified by isolating the corresponding plasmid and then analyzing the base sequence. In the two-hybrid system, a protein that is one of the test subjects and that is a target of a search may be referred to as a target protein, a prey protein, or a fish protein; and a protein that serves as a partner of the target protein may be referred to as a bait protein.

Moreover, some modified systems of the two-hybrid system have been developed. It is thought that different two-hybrid systems have to be used to identify, detect, and assay various interactions observed in biological systems, and in fact, other two-hybrid techniques have evolved, enabling investigation of protein-protein interactions in separate organisms and/or different cell compartments.

With the two-hybrid system, procedures from providing a test protein whose interaction is to be determined to a screening stage of a protein interaction can be promptly carried out by a molecular biological technique in vivo, making it possible to save the trouble of isolating/purifying and subjecting the protein of interest to a test while retaining the physiological activity of the protein. Moreover, the two-hybrid system is also advantageous in being able to readily isolating the corresponding nucleic acid sequence that encodes the interaction partner. That is, it seems that the use of the two-hybrid system in detecting a protein interaction is advantageous in terms of promptness, ease, cost, and the like.

In the present invention, an intrinsically disordered protein or a disordered sequence region thereof is regarded as a target protein (fish protein) and a group of partner proteins of the intrinsically disordered protein are regarded as bait proteins, and it is possible to screen test substances (for example, peptidomimetic compounds) for compounds that regulate the activity of the intrinsically disordered protein or for compounds that regulate the binding activity of the intrinsically disordered protein and the partner proteins thereof.

Co-immunoprecipitation is a method for collecting a protein complex by immunoprecipitation. Further expanding this method has created a method called a "pull-down assay" that uses specific binding of a tag instead of an antigen-antibody reaction. Combining mass spectrometry with these methods enables to reveal true characteristics of an unknown protein that interacts with a known protein. In the present invention, a test substance (for example, a peptidomimetic compound) that specifically binds to an intrinsically disordered protein or a disordered sequence region thereof is collected, the structure of the compound is determined, and the compound is identified, thereby enabling screening for the compound of the present invention that specifically regulates the activity of the intrinsically disordered protein. Also, it can be used for screening for a compound that specifically inhibits binding to a partner protein that exhibits an interaction with the intrinsically disordered protein.

For a specific method for immunoprecipitation, see "Molecular Cloning, A Laboratory Manual, Second Edition" (edited by Maniatis, T., et al., Cold Spring Harbor Press edited (1989)). In short, using an immunoprecipitation test using an antibody that can bind to an antigenic domain of an identified polypeptide, preferably a FLAG (registered trademark) monoclonal antibody, M1, M2, or M5, the protein may be detected. As described above, a cell is transformed by an identified polypeptide, proliferated in a culture solution, and then lysed to give a solution of a cell-produced labeled protein-like substance. This solution is incubated with a monoclonal antibody solution, and any complex between the identified polypeptide labeled protein and the antibody formed in the cell is examined precipitatively. Next, a protein/antibody complex is isolated from the precipitate. Then, the presence of a labeled protein is determined using an ordinary analytical method such as SDS polyacrylamide gel electrophoresis and fluorography under protein/antibody complex dissociating conditions. It is also possible to identify a test compound and a peptidomimetic compound bound to the complex using mass spectrometry and HPLC.

The protein chip method is a method that uses a method for detecting an interaction, such as surface plasmon resonance. Not only a kinetic analysis of an equilibrium state but also a kinetic analysis of binding/dissociation are possible.
In addition to surface plasmon resonance, examples of usable measurement methods include evanescent field molecular imaging, a fluorescence imaging analysis, a solid-phase enzyme immunoassay, fluorescence depolarization, fluorescence correlation spectroscopy, and the like.

Surface plasmon resonance is a method in which surface plasmon is excited by a molecule that interacts at the metal/liquid interface and this plasmon is measured based on the change of the intensity of reflected light (Cullen, D.C., et al., Biosensors, 3(4), 211-225 (1987-88)). In the case where a protein-molecule interaction is measured or analyzed using this method, the C-terminus labeled protein needs to be immobilized on a solid phase by the above-described method, but the target molecule does not need to be labeled.
As a support for immobilizing the C-terminus labeled protein on a solid phase, a transparent support such as glass, on which thin film of a metal such as gold, silver, or platinum is formed, is used. As a transparent support, any support can be used insofar as it is usually used for a surface plasmon resonance apparatus, and it is generally composed of a material transparent to laser light, such as glass, and a support having a thickness of about 0.1 to 5 mm is used. A suitable thickness of a metallic thin film is about 100 to 2000 Å. Commercially available products can also be used as such solid-phase supports for surface plasmon resonance apparatuses. The C-terminus labeled protein can be immobilized on the aforementioned support by the above-described method.
Regarding this method, any method may be used for bringing the target molecule into contact with the C-terminus labeled protein insofar as the method allows both molecules to contact with each other sufficiently for interaction. Preferably, a method can be used in which the C-terminus protein immobilized on a solid phase is brought into contact with a solution in which the target molecule is dissolved in a suitable concentration in a buffer usually used biochemically.
These steps may be carried out by a commercially available surface plasmon resonance apparatus, for example, a BIAcore 2000 (manufactured by Pharmacia Biosensor). After both molecules are brought into contact with each other, measuring a chronological change of the relative intensity of the reflected light of each molecule using a surface plasmon resonance apparatus, which is known per se, enables an analysis of the interaction between the C-terminus labeled protein immobilized on a solid phase and the target molecule.
Regarding this method, in the case where a large number of analyses are carried out simultaneously, for example, a method in which multiple C-terminus labeled proteins are immobilized on the aforementioned support used for a surface plasmon resonance apparatus, which has an address for each protein; a method in which multiple target molecules are brought into contact with one C-terminus labeled protein immobilized on a solid phase; or a like method is used.

Evanescent field molecular imaging is a method described in, for example, Funatsu, T., et al., Nature, 374, 555-559 (1995), in which a molecule immobilized on a solid phase on a transparent material such as glass is brought into contact with a solution of a second molecule, and the transparent material is irradiated using a light source for, e.g., laser light at an angle such that an evanescent field is generated, and the generated evanescent light is measured or analyzed with a detector. These operations can be performed using an evanescent field fluorescence microscope, which is known per se.
In the case where protein-molecule interaction is measured or analyzed using this method, one of the C-terminus labeled protein and the target molecule needs to be immobilized on a solid phase by the above-described method. Although the target molecule does not need to be labeled if immobilized on a solid phased, the target molecule needs to be labeled with the aforementioned labeling substance if used without being immobilized on a solid phase.
As a support for immobilizing the C-terminus labeled protein or the target molecule on a solid phase, a support made of a material such as glass is used, and silica glass is preferably used. Also, a support whose surface has been ultrasonically cleaned is preferable to prevent, for example, laser light scattering.
Regarding this method, any method may be used for bringing the C-terminus labeled protein or the labeled target molecule that is not immobilized on a solid phase into contact with a molecule immobilized on a solid phase insofar as the method allows both molecules to contact with each other sufficiently for interaction. A method is preferable in which a solution is prepared by dissolving, in a suitable concentration, the C-terminus labeled protein or the labeled target molecule not immobilized on a solid phase in a buffer usually used biochemically and then the solution is added dropwise to the solid-phase surface.
After both molecules are brought into contact with each other, measuring fluorescence excited by evanescent field illumination using a detector such as a CCD camera enables identification of a molecule that interacts with the molecule immobilized on a solid phase.
Regarding this method, in the case where a large number of analyses are carried out simultaneously, for example, a method in which multiple C-terminus labeled proteins or labeled target molecules are immobilized on the aforementioned support that has addresses therefor is used.

The fluorescence imaging analysis method is a method in which a labeled molecule is brought into contact with a molecule immobilized on a solid phase, and fluorescence emitted, due to the interaction of both molecules, from the labeled molecule remaining on the immobilized molecule is measured or analyzed using a commercially available fluorescence imaging analyzer.
In the case where a protein-molecule interaction is measured or analyzed using this method, one of the C-terminus labeled protein and the target molecule needs to be immobilized on a solid phase by the above-described method. In the case where the target molecule is immobilized on a solid phase and used, a labeled molecule and an unlabeled molecule are both usable. The target molecule needs to be labeled with the aforementioned labeling substance if used without being immobilized on a solid phase.
Regarding the C-terminus labeled protein, a protein that is immobilized via a labeled portion and a protein that is immobilized at a portion other than the labeled portion can be used.
As a support for immobilizing the C-terminus labeled protein or the target molecule on a solid phase, nitrocellulose membranes and nylon membranes that are usually used for immobilizing proteins, nucleic acids, and the like, as well as plastic microplates and the like can be used.
Regarding this method, any method may be used for bringing the labeled target molecule or the C-terminus labeled protein into contact with a molecule immobilized on a solid phase insofar as the method allows both molecules to contact with each other sufficiently for interaction. A method is preferable in which a solution is prepared by dissolving the labeled target molecule or the C-terminus labeled protein in a suitable concentration in a buffer usually used biochemically and then the solution is brought into contact with the solid-phase surface.
After both molecules are brought into contact with each other, preferably, a step of washing the excessively present labeled target molecule or C-terminus labeled protein with the same buffer or the like is carried out, and a fluorescence signal emitted from the labeling substance of the target molecule or the C-terminus labeled protein remaining on the solid phase, or a mixed signal of fluorescence emitted from the labeled molecule immobilized on the solid phase and fluorescence emitted from the labeled molecule remaining on the solid phase is measured or analyzed using a commercially available imaging analyzer, thus enabling identification of a molecule that interacts with the molecule immobilized on the solid phase.
Regarding this method, in the case where a large number of analyses are carried out simultaneously, for example, a method in which multiple C-terminus labeled proteins or labeled or unlabeled target molecules are immobilized on the surface of the aforementioned solid phase having addresses therefor; a method in which one C-terminus labeled protein or labeled or unlabeled target molecule is brought into contact with multiple C-terminus labeled proteins or labeled target molecules not immobilized on a solid phased; or a like method is used. In the case where multiple C-terminus labeled proteins or labeled target molecules are brought into contact, the molecules remaining on the solid phase are obtained by dissociation using the difference in buffer concentration or the like, and the molecules can be identified by analyzing them by a known method.

Solid-phase enzyme immunoassay (enzyme linked immunosorbent assay (ELISA): Crowther, J.R., Methods in Molecular Biology, 42 (1995)) is a method in which an antibody-containing solution is brought into contact with an antigen immobilized on a solid phase, and due to the interaction of both molecules (antigen-antibody reaction), fluorescence emitted from a labeled molecule (IgG or the like) that specifically binds to the antibody remaining on the antigen immobilized on the solid phase or a signal emitted from a dye that uses the labeled molecule as a substrate is measured or analyzed using a commercially available detector (ELISA reader).
In the case where a protein-molecule interaction is measured or analyzed using this method, the C-terminal labeled protein that serves as the antigen needs to be immobilized on a solid phase by the above-described method. Also, the target molecule that serves as the antibody needs to be labeled with the aforementioned labeling substance.
As a support for immobilizing the C-terminus labeled protein that serves as the antigen on a solid phase, a plastic microplate or the like that is usually used for ELISA can also be used.
Regarding this method, any method may be used for bringing the labeled target molecule that serves as the antibody into contact with the molecule immobilized on a solid phase insofar as the method allows both molecules to contact each other sufficiently for interaction. Preferably, a method in which a solution in which the labeled target molecule is dissolved in a suitable concentration in a buffer usually used biochemically is prepared, and the solution is injected onto a microplate.
After both molecules are brought into contact with each other, preferably, a step of washing the excessively present labeled target molecule not bound to the molecule immobilized on the solid phase with the same buffer or the like is carried out, and measuring or analyzing fluorescence emitted from the labeled molecule remaining on the solid phase using a commercially available ELISA reader or the like enables identification of a molecule that interacts with the molecule immobilized on a solid phase.
Regarding this method, in the case where a large number of analyses are carried out simultaneously, for example, a method in which mutually different multiple labeled target molecules are immobilized in the respective wells of the aforementioned microplate is used.

Fluorescence polarization (Perran, J., et al., J. Phys. Rad., 1,390-401 (1926)) is a method that takes advantage of the fact that a fluorescent molecule excited by fluorescence polarization emits fluorescence on the same polarization plane when in an excited state if a steady state is maintained, but in the case where the excited molecule undergoes rotational Brownian motion or the like when in an excited state, the emitted fluorescence is on a different plane from the excitation light. Molecular motion is affected by the size of the molecule, and in the case where the fluorescence molecule is a macromolecule, there is little movement of the molecule when the molecule is in an excited state, and the emitted light retains polarization, whereas in the case of a low-molecular fluorescence molecule, the emitted light is depolarized due to a high motion speed. Accordingly, the intensity of fluorescence emitted from the fluorescence molecule excited by plane polarized light is measured on the original plane and a plane that is perpendicular thereto, and information on the mobility and the condition of the molecule is obtained from the proportion of the fluorescence intensities on both planes. According to this method, the behavior of the target molecule that interacts with a fluorescence-labeled molecule can be traced without being influenced by any impurity even when there is an impurity. This is because the behavior is measured as a change of the degree of polarization only when the fluorescence-labeled molecule and the target molecule interact with each other.
As an apparatus for carrying out this method, for example, a BECON (manufactured by Panyera) and the like are commercially available, and this method can be carried out using such apparatuses.
In the case where a protein-molecule interaction is measured or analyzed using this method, the C-terminus labeled protein and the target molecule both need to be supplied as solutions. The target molecule does not need to be labeled. A molecule that has a significantly smaller molecular weight than the C-terminus labeled protein of which interaction is to be analyzed is not suitable for this method because such a molecule does not influence the Brownian motion of the C-terminus labeled protein.
Regarding this method, as a method for bringing the target molecule into contact with the C-terminus labeled protein, any method may be used insofar as both molecules are sufficiently in contact so as to interact with each other. Preferably, the contact is achieved by a method in which a solution in which the C-terminus labeled protein is dissolved in a suitable concentration in, e.g., a buffer usually used biochemically is introduced into measurement wells of a commercially available fluorescence depolarization apparatus, and further a solution in which the target molecule is dissolved in a suitable concentration in the same buffer is introduced.
It seems possible that, in the interaction between the C-terminus labeled protein and the target molecule measured in this method, specificity is not necessarily as high as that in an antigen-antibody method, and accordingly, it is effective to quantify the extent of interaction in order to determine an optimum combination. As an indicator of the extent of interaction, for example, a value of a minimum target substance concentration may be used that gives the maximum fluorescence polarization degree to a specific concentration of the C-terminus labeled protein.
Regarding this method, in the case where a large number of analyses are carried out simultaneously, for example, a method in which mutually different multiple C-terminus labeled proteins are introduced into the respective measurement wells of the aforementioned fluorescence depolarization measurement apparatus and then a solution of a specific target molecule is introduced into the wells, or a method in which a specific C-terminus labeled protein is introduced into the wells and then solutions of mutually different multiple target molecules are introduced into the wells may be used.

Fluorescence correlation spectroscopy (FCS, Eigen, M., et al., Proc. Natl. Acad. Sci. USA, 91, 5740-5747 (1994)) is a method in which the flowing velocity, the diffusion coefficient, the volume shrinkage, and the like of particles are measured under a confocal laser scanning microscope or the like. In the present invention, a change of the translational Brownian motion of one molecule of the original labeled molecule is measured based on the interaction between the C-terminus labeled protein and the target molecule, thus enabling an interacting molecule to be determined.
Specifically, sample particles are excited by excitation light, a portion of a sample solution volume emits fluorescence, and emitted light is measured to obtain the proportion of photon. This value changes in accordance with the number of particles present in a space volume observed at a specific time. The aforementioned various parameters can be calculated from changes of the signal using an autocorrelation function. An apparatus for performing FCS is also commercially available from, for example, Carl Zeiss, and in this method also, analyses can be performed using such apparatuses.
In the case where a protein-molecule interaction is measured or analyzed using this method, the C-terminus labeled protein and the target molecule both need to be supplied as solutions. The target molecule does not need to be labeled. A molecule that has a significantly smaller molecular weight than the C-terminus labeled protein of which interaction is to be analyzed is not suitable for this method because such a molecule does not influence the Brownian motion of the C-terminus labeled protein.
Regarding this method, as a method for bringing the target molecule into contact with the C-terminus labeled protein, any method may be used insofar as both molecules are sufficiently in contact so as to interact with each other. Preferably, the contact is achieved by a method in which a solution in which the C-terminus labeled protein is dissolved in a suitable concentration in, e.g., a buffer usually used biochemically is introduced into measurement wells of a commercially available FCS apparatus, and further a solution in which the target molecule is dissolved in a suitable concentration in the same buffer is introduced.
Regarding this method, in the case where a large number of analyses are carried out simultaneously, for example, a method in which mutually different multiple C-terminus labeled proteins are introduced into the respective measurement wells of the aforementioned FCS measurement apparatus and then a solution of a specific target molecule is introduced into the wells, or a method in which a specific C-terminus labeled protein is introduced into the wells and then solutions of mutually different multiple target molecules are introduced into the wells may be used.

In the invention of the present application, the above-mentioned methods can be used for measuring an intermolecular interaction between an intrinsically disordered protein or a disordered sequence region thereof and a test substance.
Regarding a conformational analysis, in the case where the specific structure of a complex is determined using X-ray diffraction, NMR, or the like, a crystal is formed in the presence of an intrinsically disordered protein or a disordered sequence region thereof and a test substance, and an X-ray analysis is carried out, thus enabling their binding to be analyzed. Also, a solution in which an intrinsically disordered protein or a disordered sequence region thereof and a test substance are present is subjected to NMR measurement, thus enabling an analysis of binding between the disordered sequence region and the test substance.
A crosslinking method crosslinks complex protein molecules with a low molecular weight compound and fixes the molecules, thus attaining a crosslink in the presence of an intrinsically disordered protein or a disordered sequence region thereof and a test substance, and enabling detection of an interaction between the disordered sequence region and the test substance.

In the case where an interaction between a disordered sequence region of an intrinsically disordered protein and a test substance is detected, or in the case where a decrease of an interaction between a disordered sequence region of an intrinsically disordered protein and a partner protein is detected, the test substance subjected to the measurement is identified as a compound that regulates the activity of the intrinsically disordered protein, or as a compound that regulates the binding activity of the intrinsically disordered protein and the partner protein thereof.
Therefore, according to the screening method of the present invention, an activity regulating agent for an intrinsically disordered protein or an activity regulating agent for binding of an intrinsically disordered protein and a partner protein thereof can be obtained.

The present invention also provides a pharmaceutical agent containing a compound obtained by the above-described screening method.
A compound that binds to a disordered sequence region of an intrinsically disordered protein (for example, a compound obtained by the screening method of the present invention) is effective as an activity regulating agent for an intrinsically disordered protein, and the compound, depending on its type, can be prepared as a pharmaceutical composition using pharmaceutically acceptable carriers, excipients, and/or other additives that are usually used for formulating such compounds into pharmaceutical agents. Examples of administration forms include oral administration using tablets, pills, capsules, granules, subtle granules, powders, oral solutions; injections such as intravenous injections (including infusions), intramuscular injections, and subcutaneous injections; and parenteral administration using suppositories, percutaneously administered agents, and transmucosaly administered agents. In particular, parenteral administration such as intravenous injection is preferable for peptides that are digested in the stomach.

Regarding a solid pharmaceutical composition for oral administration, one or more active substances may be mixed with at least one inert diluent such as lactose, mannitol, glucose, microcrystalline cellulose, hydroxypropylcellulose, starch, polyvinyl pyrrolidone, or magnesium aluminometasilicate. The composition may contain, according to an ordinary method, additives other than the inert diluent, such as lubricants, disintegrating agents, stabilizers, solubilizers, or solubilizing aids. Tablets and pills may be sugar-coated or coated with a film of a gastrosoluble or enterosoluble substance as necessary.

A liquid pharmaceutical composition for oral administration may contain opacifiers, solubilizing agents, suspending agents, syrups, or elixirs, and may also contain generally used inert diluents such as purified water or ethanol. The composition may contain, additives other than inert diluents, such as wetting agents, suspending agents, sweeteners, flavorings, or preservatives.

A parenteral injection may contain sterile aqueous or nonaqueous liquid agents, suspending agents, or opacifiers. Water-soluble solutions or suspensions may contain, for example, injectable distilled water or physiological saline as a diluent. Nonaqueous solutions or suspensions may contain, for example, propylene glycol, polyethylene glycol, vegetable oil (such as olive oil), alcohols (such as ethanol), polysorbate 80, or the like as a diluent. The aforementioned compositions may further contain wetting agents, emulsifiers, dispersants, stabilizers, solubilizers, solubilizing aids, preservatives, or the like. The aforementioned compositions may be sterilized by, for example, filtration through a bacteria-retaining filter, inclusion of a bactericide, or irradiation. Alternatively, it is possible that a sterile solid composition is produced, and used after being dissolved in sterile water or other sterile injection media when used.

Dosage may be suitably determined in light of the strength of the activity of the active substance, i.e., the substance obtained by the method for screening of the present invention, the symptom, the age of the administration target, the sex, or the like.

### Examples

The present invention shall be described in detail below by way of examples, but it should be understood that the present invention is not limited thereto.

### Example 1

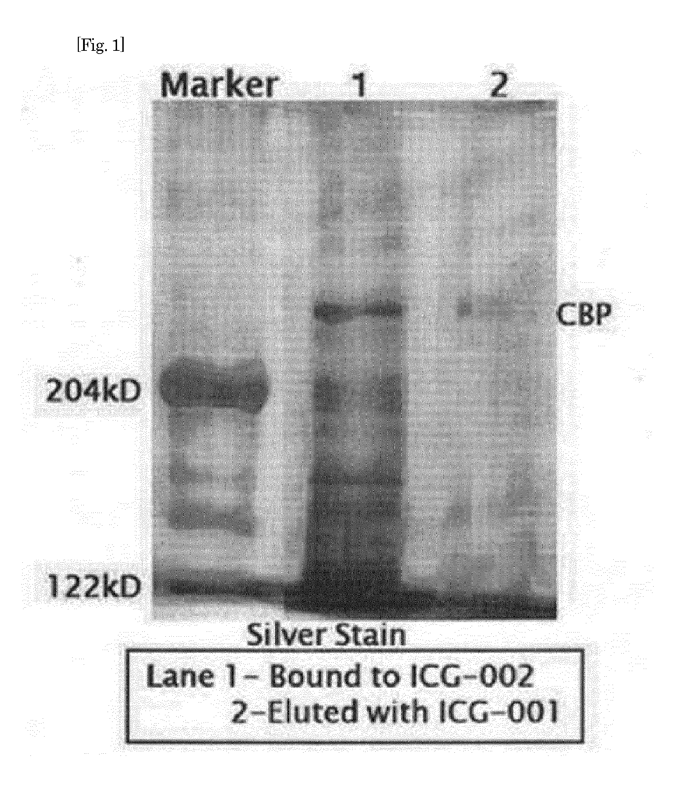

An outline of an experiment described in an already published paper (Proc Natl Acad Sci USA. 2004 Aug 24; 101(34): 12682-7) is presented below to demonstrate the present invention. While no inventive concept of the present invention is disclosed in the paper, the content of the paper constitutes the present specification. The paper merely reveals that a peptidomimetic compound ICG-001 simply binds to CBP, and in particular binds to the N-terminus sequence of CBP, but does not binds to p300. However, it is clear that the paper establishes the technical concept of the present invention.
ICG-001 is shown in Formula 1, and biotinylated ICG-001 is shown in Formula 2 (ICG-002). Fig. 1 shows results of an analysis by immunoprecipitation using these compounds. Lane 1 is for a nuclear extract of an SW480 cell (human colon cancer cell line) incubated with ICG-002 bound to streptavidin-agarose beads, and Lane 2 is for a nuclear extract pulled down with ICG-001.

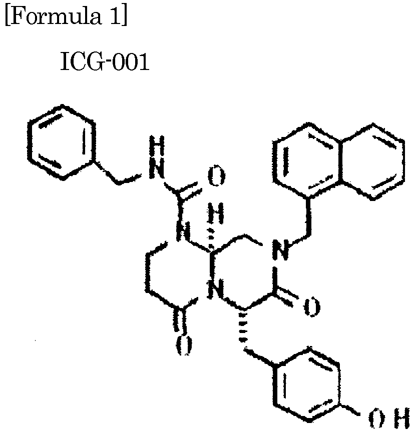

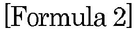

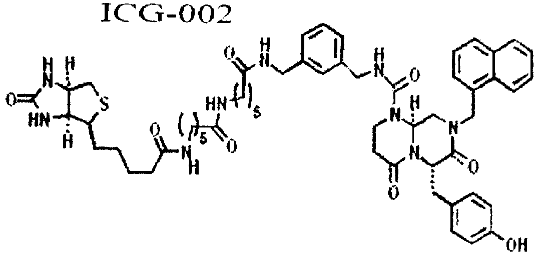

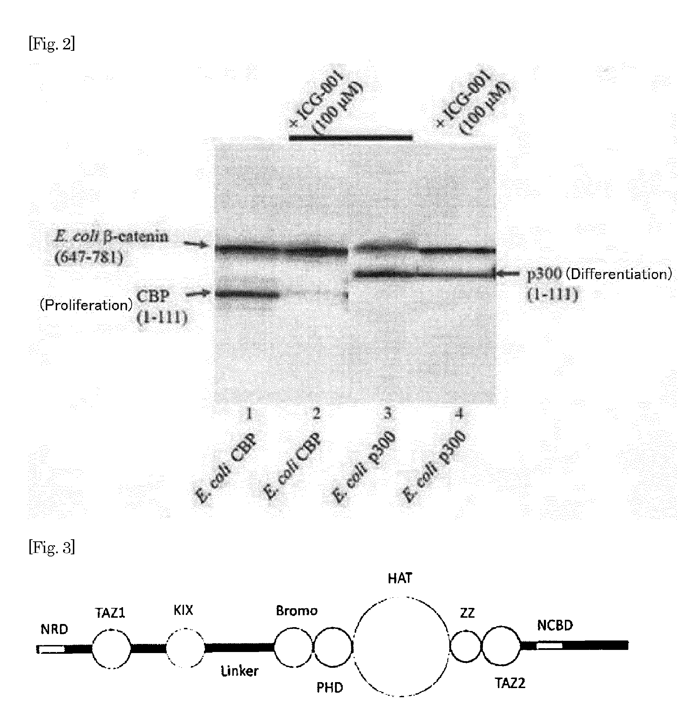

When CBP (1-111) (disordered sequence region) as well as ß-catenin and p300 (1-111) (disordered sequence region) expressed in E. coli were pulled down according to immunoprecipitation, CBP (1-111) became separated due to the addition of ICG-001, whereas ICG-001 did not bind to P300, which is highly analogous to CBP (see Fig. 3).

### Example 2

¹⁵N-labeled CBP (1-111) (disordered sequence region) was expressed E. coli and dissolved in a buffer (NaP 25 mM·pH 6.8, NaCl 50 mM, 0.02% NaN3), and 300 µl thereof was introduced into an NMR sample tube. Moreover, a D₂O solution of ICG-001 was added so as to attain an ICG-001 concentration of 5 µM or 15 µM. Solutions prepared in this manner as NMR samples were subjected to an NMR analysis. In the NMR analysis, the measurement and analysis was carried out to give a 2D spectrum, with the X axis indicating C-13 data and the Y axis indicating N-15 data.

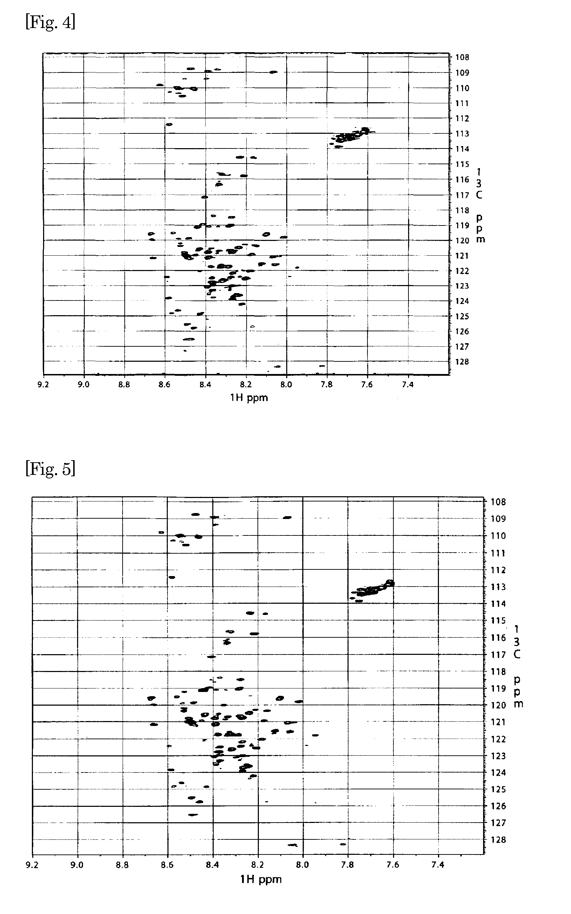

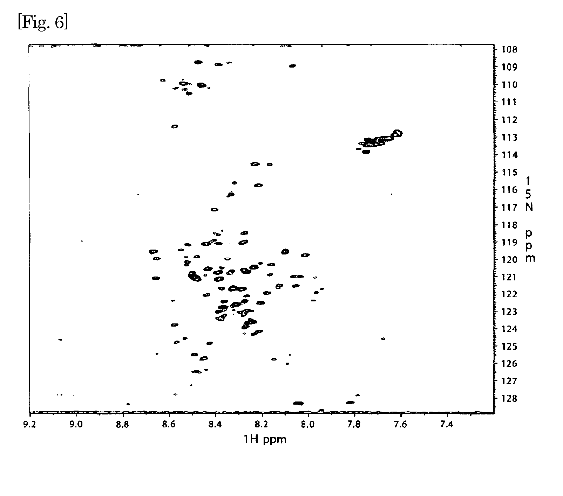

Fig. 4 shows results of CBP-111, Fig. 5 shows results of CBP-111 with which 5 µM of ICG-001 was concomitantly present, and Fig. 6 shows results of CBP1-111 with which 15 µM of ICG-001 was concomitantly present. As a result, CBP1-111 showed a typical peak of a naturally disordered protein (see Fig. 4), and a change of a peak due to the addition of ICG-001 was observed (see Figs. 5 and 6). The results establish that there is a clear interaction between CBP1-111 and ICG-001.

### [Industrial Applicability]

The present invention provides a compound that binds to a disordered sequence region of an intrinsically disordered protein, an activity regulating agent for an intrinsically disordered protein that contains the compound, a pharmaceutical containing the compound, and a method for screening for the compound. These are useful because binding of the compound to an intrinsically disordered protein alters the structure of the disordered sequence domain, making it impossible for the protein to bind to a partner protein, which should occur in a natural state, and thus a compound that can specifically regulate a biological reaction that occurs in a natural state can be provided. Moreover, a groundbreaking pharmaceutical product based on a novel mechanism can be provided.

## Claims

1. A method for screening for an activity regulating agent for an intrinsically disordered protein, comprising the steps of:
(1) bringing the protein or a disordered sequence region of the protein into contact with a test substance, and
(2) detecting an interaction between the disordered sequence region of the protein and the test substance.

2. A method for screening for an activity regulating agent for binding between an intrinsically disordered protein and a partner protein thereof, comprising the steps of:
(1) bringing the protein or a disordered sequence region of the protein into contact with a test substance, and
(2) detecting an interaction between the disordered sequence region of the protein and the test substance.

3. A method for screening for an activity regulating agent for binding between an intrinsically disordered protein and a partner protein thereof, comprising the steps of:
(1) bringing the protein or a disordered sequence region of the protein, the partner protein, and a test substance into contact with each other, and
(2) detecting an interaction between the disordered sequence region of the protein and the test substance.

4. A method for screening for an activity regulating agent for binding between an intrinsically disordered protein and a partner protein thereof, comprising the steps of
(1) bringing the protein or a disordered sequence region of the protein, the partner protein, and a test substance into contact with each other, and
(2) detecting an interaction between the disordered sequence region of the protein and the partner protein.

5. The screening method according to any of claims 1 to 4, wherein the test substance is a peptidomimetic compound.

6. The screening method according to any of claims 1 to 5, wherein the intrinsically disordered protein is a transcription regulatory factor, a protein of a signal transduction system, or an intracellular protein.

7. The screening method according to claim 1, wherein detection of interaction is carried out according to a two-hybrid method (Y2H), co-immunoprecipitation, a protein chip method, a conformational analysis, far-Western blotting, a crosslinking method, or fluorescence quenching.

8. A compound obtained by a screening method of claims 1 to 8.

9. A compound that binds to a disordered sequence region of an intrinsically disordered protein.

10. An activity regulating agent for an intrinsically disordered protein, comprising a compound that binds to a disordered sequence region of the intrinsically disordered protein.

11. A method for regulating activity of an intrinsically disordered protein using a compound that binds to a disordered sequence region of the intrinsically disordered protein.

12. A pharmaceutical comprising a compound that binds to a disordered sequence region of an intrinsically disordered protein.
